# EUROPÄISCHE PATENTSCHRIFT

(11) **EP 2 086 499 B1**
(45) Veröffentlichungstag und Bekanntmachung des Hinweises auf die Patenterteilung: **02.02.2011**
(21) Anmeldenummer: 07818279.7
(22) Anmeldetag: 20.09.2007
(51) Int. Cl.: A61K 8/44, A61Q 19/00

(54) **KOSMETISCHE ODER DERMATOLOGISCHE ZUBEREITUNGEN MIT EINEM GEHALT AN 2-ISOPROPYL-5- METHYL-CYCLOHEXANCARBONYL-D-ALANINMETHYLESTER UND EINEM ODER MEHREREN HAUTBEFEUCHTUNGSMITTELN**
COSMETIC OR DERMATOLOGICAL PREPARATIONS WITH A CONTENT OF 2-ISOPROPYL-5- METHYL-CYCLOHEXANECARBONYL-D-ALANINE METHYL ESTER AND ONE OR MORE SKIN MOISTURISING AGENTS
PRÉPARATIONS COSMÉTIQUES OU DERMATOLOGIQUES COMPRENANT DE L'ESTER MÉTHYLIQUE DE LA 2-ISOPROPYL-5- METHYL-CYCLOHEXANCARBONYL-D-ALANINE ET UN OU PLUSIEURS AGENTS D'HYDRATATION DE LA PEAU

(30) Priorität: 29.09.2006 DE 102006047164
(43) Veröffentlichungstag der Anmeldung: 12.08.2009
(73) Patentinhaber: Beiersdorf AG, 20253 Hamburg (DE)
(72) Erfinder: ECKERT, Julia, 22085 Hamburg (DE); KOLBE, Ludger, 21255 Dohren (DE); NEUFANG, Gitta, 20149 Hamburg (DE)
(86) Internationale Anmeldenummer: PCT/EP2007/008187
(87) Internationale Veröffentlichungsnummer: WO 2008/040453

(56) Entgegenhaltungen:
- EP-A- 1 639 993
- WO-A-02/30367
- WO-A-02/32381
- WO-A-2006/103401
- DE-A1- 2 205 255
- US-A1- 2006 171 912
- WATSON H R ET AL: "NEW COMPOUNDS WITH THE MENTHOL COOLING EFFECT" JOURNAL OF THE SOCIETY COSMETIC CHEMISTS, SOCIETY OF COSMETIC CHEMISTS,, US, Bd. 29, Nr. 4, 1978, Seiten 185-200, XP009045124 ISSN: 0037-9832

## Beschreibung

Die vorliegende Erfindung betrifft kosmetische oder dermatologische Zubereitungen mit einem Gehalt an 2-Isopropyl-5-Methyl-Cyclohexancarbonyl-D-Alaninmethylester und einem oder mehreren Hautbefeuchtungsmitteln.

Die vorliegende Erfindung betrifft in einer besonderen Ausführungsform kosmetische oder pharmazeutische Zubereitungen mit vermindertem Klebrigkeitsgefühl, Verfahren zu ihrer Herstellung sowie die Verwendung von Wirkstoffen zur Herabminderung des Klebrigkeitsgefühles kosmetischer Zubereitungen.

Die vorliegende Erfindung betrifft in einer ganz besonderen Ausführungsform kosmetische und dermatologische Zubereitungen mit langanhaltender kühlender Wirkung, insbesondere hautpflegende kosmetische und dermatologische Zubereitungen.

Die vorliegende Erfindung betrifft in einer ganz besonderen Ausführungsform kosmetische und dermatologische Zubereitungen mit verminderter Hautreizung.

Die Kühlwirkung kosmetischer Zubereitungen beruht bisher auf zwei Grundprinzipien:

Einsatz von Komponenten die sich nach topischer Applikation gasförmig verflüchtigen und die hierfür erforderlich Energiemenge, die sog. Verdampfungsenthalpie, zu einem Großteil der Hautoberfläche entnehmen. Es werden daher in entsprechenden nicht okklusiven Kosmetika geeignete flüssige Komponenten eingesetzt. Als besonders geeignet hat sich hier Ethanol erwiesen, daneben zeigen Formulierungen mit hohem Wassergehalt ebenfalls eine deutliche Kühlwirkung.

Einsatz von sogenannten "Cooling Agents", die mit den Wärmerezeptoren der Haut in Wechselwirkung treten und somit ein Kälteempfinden auslösen, ohne eine meßbare physikalische Abkühlung zu generieren. Hierfür kommen insbesondere Menthol und diverse Mentholderivate (Frescolate, Physcool, Questice L, etc.) zum Einsatz. Insbesondere hohe Ethanolgehalte sowie Menthol und seine Derivate sind, neben dem irritativen Potential, insbesondere aufgrund ihres deutlichen Eigengeruchs für zahlreiche Einsatzzwecke unter olfaktorischen Gesichtspunkten nicht geeignet. Häufig genug bewirken solche Substanzen darüberhinaus gleichzeitig eine Durchblutungssteigerung, die im Gegenteil ein Wärmegefühl hervorruft.

In der Literatur werden beispielsweise ionische Verbindungen, insbesondere Ammoniumsalze, als kühlende Agenzien beschrieben. Als kühlende Zubereitungen werden auch verbreitet isopropanolische Gele mit Campher- und Mentholzusatz angewandt.

Die Anwendung dieser Substanzen, namentlich auf gereizter Haut, ist jedenfalls problematisch. Darüber hinaus sind viele dieser Verbindungen schlecht wasserlöslich. Ihre Verwendung ist folglich auf wenige Kosmetika und Dermatika beschränkt.

EP-1 639 993 offtenbart kosmetische Zubereitungen, die N-substituierte-p-Menthan-3-carboxamid Derivate enthalten.

WO-02/30 367, WO-02/32 381 und US-2006/0171912 offenbaren kosmetiche Zubereitungen, denen Ethylmenthan carboxamid zugesetzt werden kann.

DE-2 205 255 und H.R. Watson et al., J. Soc Cosmet, Chem., 29, 1978, 187-200 offenbaren 2-Isopropyl-5-methyl-cyclohexancarbonyl-D-Alanin ethyl ester als kühlendes Mittel in kosmetischen Zubereitungen.

Aufgabe der vorliegenden Erfindung war es also, pflegende kosmetische und medizinische Zubereitungen zur Verfügung zu stellen, die nicht die Nachteile des Standes der Technik haben, insbesondere solche, welche, auf der Haut oder Schleimhäuten angewandt, befeuchtend und/oder kühlend wirken.

Die DE 43 12 656 beschreibt die Verwendung kosmetisch oder pharmazeutisch unbedenklicher Substanzen mit positiver Lösungsenthalpie in kosmetischen oder medizinischen topischen Zubereitungen, **dadurch gekennzeichnet, dass** die Substanz oder die Substanzen in den Zubereitungen in einem weitgehend wasserfreien Medium vorliegen und/oder durch eine stoffliche Barriere von einem wasserhaltigen Medium abgeschirmt werden.

Obwohl dieses Vorgehen grundsätzlich zu kosmetisch befriedigenden Zubereitungen führen kann, sind diese jedoch galenisch äußerst aufwendig herzustellen.

Es war also die Aufgabe, der vorliegenden Erfindung, den Übelständen des Standes der Technik abzuhelfen und kühlende kosmetische oder dermatologische Zubereitungen zur Verfügung zu stellen, welche einfach herzustellen sind, keine Reizwirkung auf Haut oder Schleimhäute ausüben - beispielsweise unangenehmes Kribbeln oder Juckreiz - sowie bei bestimmungsgemäßer Anwendung angenehme Kühlung spenden.

Der Einsatz von Hautbefeuchtungsmittel - gerne verwendet werden mehrwertige Alkohole wie Glycerin - ist im allgemeinen erwünscht. Ein Nachteil von Zubereitungenes Standes der Technik ist aber, dass diese - sofern Polyolmengen eingesetzt werden, welche in bezug auf die Hautbefeuchtung eine gewisse Wirksamkeit zeigen - eine deutliche Klebrigkeit aufweisen und sich nur schlecht auf der Haut verteilen lassen.

Um die Klebrigkeit polyolhaltiger O/W-Emulsionen zu verringern und ein leichtes Hautgefühl zu erreichen (leicht verteilbar, kaum Rückstand, schnell einziehend), wird üblicherweise mit sehr dünnflüssigen Ölen gearbeitet (d. h. mit Ölen mit einer Viskosität von 1 bis 15 mPa.s), die ferner eine sehr hohe Spreitfähigkeit (800 bis 1200 mm2/10 min) aufweisen.

Es war also Aufgabe der Erfindung, gute Hautbefeuchtung zu bewerkstelligen, ohne dass die kosmetische Anmutung der Zubereitungen darunter leidet.

Ferner war eine Aufgabe der vorliegenden Erfindung, Zubereitungen zur Verfügung zu stellen, welche den Zustand der Haut deutlich verbessern, insbesondere die Hautrauhigkeit vermindern.

Aufgabe war daher, all diesen den Nachteilen des Standes der Technik Abhilfe zu schaffen. Insbesondere sollten Produkte mit verringerter Klebrigkeit bzw. Schmierigkeit zur Verfügung gestellt werden. Produkte auf dem Gebiete der pflegenden Kosmetik, der dekorativen Kosmetik und der pharmakologischen Galenik sollten gleichermaßen von den geschilderten Nachteilen des Standes der Technik befreit werden.

Weiterhin war es eine Aufgabe der Erfindung, kosmetische Grundlagen für kosmetische Zubereitungen zu entwickeln, die sich durch gute Hautverträglichkeit auszeichnen.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen, aber auch ausgesprochen medizinisch-pharmazeutische Darreichungsformen geschaffen werden, zum Beispiel Zubereitungen gegen Akne und andere Hauterscheinungen.

Erfindungsgemäß gelöst werden diese Aufgaben durch kosmetische oder dermatologische Zubereitungen mit einem Gehalt an 2-Isopropyl-5-Methyl-Cyclohexancarbonyl-D-Alaninmethylester und einem oder mehreren Hautbefeuchtungsmitteln gemäß Anspruch 1.

Erfindungsgemäße Zubereitungen zeichnen sich durch hervorragende, angenehme Kühlwirkung und elegante kosmetische Anmutung aus. Sie sind nicht unangenehm Klebrig und stabil gegen physikalische Zersetzung wie beispielsweise Auf- oder Abrahmen ihrer Bestandteile.

2-Isopropyl-5-Methyl-Cyclohexancarbonyl-D-Alaninmethylester zeichnet sich durch folgende Struktur aus:

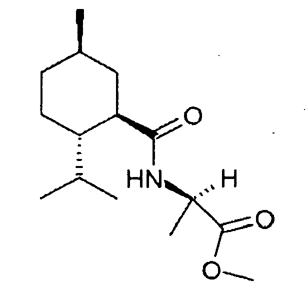

Die Synthese von 2-Isopropyl-5-Methyl-Cyclohexancarbonyl-D-Alaninmethylester [synonom: (R)-2-[((1R,2S,5R)-2-isopropyl-5-methyl-cyclohexancarbonyl)-amino]-propionsäuremethylester kann nach folgender Vorschrift erfolgen:

1,0 g D-Alaninmethylesterhydrochlorid (von Aldrich Chemical Co erhalten) wurde in 28 ml diethylether und 1 ml doppeltdestillierten Wassers aufgelöst und auf 0 °C gekühlt. Eine Spatelspitze des Katalysators Diaminopyrimidin wurde hinzugefügt. 1,62 ml p-Menthylchlorid wurden tropfenweise hinzugefügt, gefolgt von 2 ml Triethylamin. Flocken eines falrblosen Niederschlages bildeten sich in der Mischung, die über Nacht bei Raumtemperatur gerührt wurde. Der Niederschlag wurde in Ethylacetat gelöst, mit doppelt destilliertem Wasser gewaschen und über Natriumsulfat getrocknet. Die organische Phase wurde unter vermindertem Druck verdampft, wobei 2 g Endprodukt erhalten wurde, welches bei Raumtemperatur kristallisierte. Die erwartete Molekularmasse und Struktur wurden mit Massenspektrometer bzw. anhand des NMR-Spektrums bestätigt.

Hautbefeuchtungsmittel werden auch oft als sog. "Moisturizer" bezeichnet.. Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Methylpropandiol, Hexandiol, Oktandiol, Taurin, Natriumchlorid, Milchsäure und/oder Lactate, insbesondere Natriumlactat, Butylenglykol, Propylenglykol, Biosaccaride Gum-1, Ethylhexyloxyglycerin, Pyrrolidoncarbonsäure, Hyaluronsäure und Harnstoff.

Bevorzugt enthalten kosmetische oder dermatologische Zubereitungen gemäß der Erfindung 0,001 - 10 Gew.-%, besonders bevorzugt 0,01 - 1 Gew.-%, an einem oder mehreren Hautbefeuchtungsmitteln, bezogen auf die Gesamtzusammensetzung der Zubereitungen.

Die kosmetischen oder dermatologischen Zubereitungen können erfindungsgemäß wie üblich zusammengesetzt sein und zur Behandlung, der Pflege und der Reinigung der Haut und/oder der Haare und als Schminkprodukt in der dekorativen Kosmetik dienen.

Sie enthalten bevorzugt 0,001 Gew.-% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an 2-Isopropyl-5-Methyl-Cyclohexancarbonyl-D-Alaninmethylester.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch erfindugsgemäß vorteilhaft, Folsäure und/oder deren Derivate in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, Folsäure und/oder deren Derivate in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Siliconderivate.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Siliconöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die wäßrige Phase der erfindungsgemäßen Zubereitungen enthält gegebenenfalls vorteilhaft Alkohole, 1,2-Propandiol, andere Diole oder Polyole niedriger C-Zahl, sowie deren Ether, vorzugsweise Ethanol, Isopropanol, Ethylenglykol, Ethylenglykolmonoethyl- oder -monobutylether, Propylenglykolmonomethyl, -monoethyl- oder -monobutylether, Diethylenglykolmonomethyl-oder -monoethylether und analoge Produkte, sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, Gewichtsprozente, auf das Gewicht und die Gesamtmenge bzw. auf das Gesamtgewicht der Zubereitungen bezogen.

| **Beispiel 1** | **Gew.-%** |
|---|---|
| O/W-Creme | |
| Glycerylstearat | 1 |
| Stearinsäure | 3 |
| Stearylalkohol | 2 |
| Cetylalkohol | 2 |
| C₁₂₋₁₅ Alkylbenzoat | 2 |
| Caprylsäure/Caprinsäuretriglyceride | 2 |
| Macadamiaöl | 1 |
| Myristylmyristat | 2 |
| Dimethicon | 2 |
| Hydrierte Kokosfettglyceride (Hydrogenated Coco Glycerides) | 1 |
| Tocopherylacetat | 1 |
| 2-Isopropyl-5-Methyl-Cyclohexancarbonyl-D-Alaninmethylester | 0,5 |
| Kreatin | 0,1 |
| Ubichinon (Q10) | 0,03 |
| Phenoxyethanol | 0,4 |
| p-Hydroxybenzoesäurealkylester (Paraben) | 0,3 |
| lodopropinylbutylcarbamat | 0,02 |
| Cyclodextrin | 0,3 |
| Iminodisuccinat | 0,2 |
| Carragenan | 0,3 |
| Glycerin | 5 |
| Butylenglycol | 3 |
| Methylpropandiol | 1 |
| Additive (SiO₂, Talkum) | 0,5 |
| Parfüm | q.s. |
| Wasser | ad 100 |

| **Beispiel 2** | **Gew.-%** |
|---|---|
| After Sun Gel | |
| Cetylalkohol | 2 |
| Sheabutter | 1 |
| Caprylsäure/Caprinsäuretriglyceride | 2 |
| Octyldodecanol | 1 |
| Dicaprylylcarbonat | 5 |
| Dimethicon | 2 |
| Polydecen | 2 |
| Methylpalmitat | 3 |
| 2-Isopropyl-5-Methyl-Cyclohexancarbonyl-D-Alaninmethylester | 0.75 |
| Natriumascorbylphosphat | 0,05 |
| Iminodisuccinat | 0,2 |
| Ethanol | 2 |
| Butylenglykol | 4 |
| p-Hydroxybenzoesäurealkylester (Paraben) | 0,4 |
| Vernetztes Alkylacrylat (Alkylacrylate Crosspolymer) | 0,2 |
| Carragenan | 0,3 |
| Glycerin | 5 |
| Parfüm | q.s. |
| Wasser | ad 100 |

| **Beispiel 3** | **Gew.-%** |
|---|---|
| After Shave Gel | |
| Triceteareth-4-Phsophate | 2,0 |
| Cyclometicone | 2,0 |
| Octyldodecanol | 1,0 |
| Dicaprylylcarbonat | 3,0 |
| Methylpalmitat | 2,0 |
| 2-Isopropyl-5-Methyl-Cyclohexancarbonyl-D-Alaninmethylester | 0.7 |
| Allantoin | 0,1 |
| Panthenol | 0,5 |
| Polyvinylpyrrolidon | 0,2 |
| Ethanol | 5,0 |
| Phenoxyethanol | 0,5 |
| p-Hydroxybenzoesäurealkylester (Paraben) | 0,4 |
| Carbopol Ultrez 10 | 0,1 |
| Distärkephosphat | 1,0 |
| Butylenglycol 3,0 | |
| Glycerin | 4,0 |
| Parfüm | q.s. |
| Wasser | ad 100 |

| **Beispiel 4** | **Gew.-%** |
|---|---|
| O/W Creme | |
| Glycerylsterat | 2,5 |
| PEG-40-Stearät | 1 |
| Cetearylalkohol | 2 |
| Hydrierte Kokosfettglyceride (Hydrogenated Coco Glycerides) | 1 |
| Myristylmyristate | 2 |
| C₁₂₋₁₅Alkylbenzoat | 4 |
| Caprylsäure/Capririsäuretriglyceride | 2 |
| Octyldodecanol | 1 |
| Dicaprylylcarbonat | 3 |
| Ethylhexylcyariodiphenylacrylat (Octocrylen) | 5 |
| 2-Hydroxy-4-Methoxy- benzophenon (Oxybenzone) | 3 |
| Ubichinon (Q10) | 0,03 |
| 2-Isopropyl-5-Methyl-Cyclohexancarbonyl-D-Alaninmethylester | 0,5 |
| Alpha-Glucosylrutin | 0,1 |
| Citronensäure | 0,8 |
| Phenoxyethanol | 0,5 |
| p-Hydroxybenzoesäurealkylester (Paraben) | 0,4 |
| lodopropinylbutylcarbamat | 0,05 |
| 2-Ethylhexylglycerin | 0,5 |
| Acrylates/ Steareth-20 Itaconate Copolymer | 0,2 |
| Nylonmikropartikel | 1 |
| Glycerin | 10 |
| Additive (Distärkephosphat, EDTA, BHT) | 0.5 |
| Biosaccharide Gum | 2 |
| Wasser | ad 100 |

| **Beispiel 5** | **Gew.-%** |
|---|---|
| O/W Creme | |
| Polyglyceryl-3-Methylglucosedistearat | 3 |
| Cetylalkohol | 3 |
| C₁₂₋₁₅ Alkylbenzoat | 2 |
| Butylenglycol Dicaprylat/Dicaprat | 2 |
| Caprylsäure/Caprinsäuretriglyceride | 2 |
| Hydriertes Polydecen | 1 |
| Dimethylpolysiloxan (Dimethicon) | 1 |
| Isodecylneopentanoat | 4 |
| Bis-Ethylhexyloxyphenolmethoxyphenyltriazin | 1 |
| Ethylhexylmethoxycinnamat | 5 |
| 2-Isopropyl-5-Methyl-Cyclohexancarbonyl-D-Alaninmethylester | 0, 5 |
| Natriumascorbylphosphat | 0,1 |
| EDTA | 0,2 |
| Phenoxyethanol | 0,4 |
| Iodopropinylbutylcarbamat | 0,05 |
| p-Hydroxybenzoesäurealkylester (Paraben) | 0,4 |
| Carbomer 980 | 0,1 |
| Hydroxypropylstärkephosphatester | 0,2 |
| Glycerin | 7.5 |
| Additive (Distärkephosphat, Talkum, BHT) | 0,2 |
| Hyaluronsäure | 0.3. |
| Wasser | ad 100 |

## Patentansprüche

1. Kosmetische oder dermatologische Zubereitungen mit einem Gehalt an 2-Isopropyl-5-Methyl-Cyclohexancarbonyl-D-Alaninmethylester und einem oder mehreren Hautbefeuchtungsmitteln
mit Ausnahme von 0,1 und 0,5 -prozentigen (Gewichtsprozent) Lösungen des 2-Isopropyl-5-Methyl-Cyclohexancarbony-D-Alaninmethylesters in Lösungen von 10 % Propylenglycol in 90 % Wasser.

2. Zubereitungen nach Anspruch 1, **dadurch gekennzeichnet, daß** das der oder die Hautbefeuchtungsmitteln gewählt wird oder werden aus der Gruppe Glycerin, Methylpropandiol, Hexandiol, Oktandiol, Taurin, Natriumchlorid, Milchsäure und/oder Lactate, insbesondere Natriumlactat, Butylenglykol, Propylenglykol, Biosaccaride Gum-1, Ethylhexyloxyglycerin, Pyrrolidoncarbonsäure, Hyaluronsäure und Harnstoff.

3. Zubereitungen nach einem der vorstehenden Ansprüche, **dadurch gekennzeichnet, daß** sie 0,001 Gew.-% bis 10 Gew.-%, bevorzugt 0,01 Gew.-% bis 1,0 Gew.-%, insbesondere 0,05 - 0,5 Gew.-%, bezogen auf das Gesamtgewicht der Zubereitungen, an 2-Isopropyl-5-Methyl-Cyclohexancarbonyl-D-Alaninmethylester enthalten.

4. Zubereitungen nach einem der vorstehenden Ansprüche, **dadurch gekennzeichnet, daß** sie 0,001 Gew.-% bis 20 Gel.-%, bevorzugt 0,01 Gew.-% bis 10 Gew.-°%, insbesondere 0.1 - 10 Gew.-%, bezogen auf das Gesamtgewicht der Zubereitungen, an einem oder mehreren Hautbefeuchtungsmitteln enthalten.

## Claims

1. Cosmetic or dermatological preparations with a content of 2-isopropyl-5-methylcyclohexanecarbonyl-D-alanine methyl ester and one or more skin moisturizing agents
with the exception of 0.1 and 0.5 per cent strength (per cent by weight) solutions of 2-isopropyl-5-methyl-cyclohexanecarbnnyl-D-alanine methyl ester in solutions of 10% propylene glycol in 90% water.

2. Preparations according and Claim 1, **characterized in that** the skin moisturizing agent or agents is/are selected from the group consisting of glycerol, methylpropanediol, hexanediol, octanediol, taurine, sodium chloride, lactic acid and/or lactates, in particular sodium lactate, butylene glycol, propylene glycol, biosaccharide gum-1, ethylhexyloxyglycerol, pyrrolidonecarboxylic acid, hyaluronic acid and urea.

3. Preparations according to one of the preceding claims, **characterized in that** they comprise 0.001% by weight to 10% by weight, preferably 0.01% by weight to 1.0% by weight, in particular 0.05-0.5% by weight, based on the total weight of the preparations, of 2-isopropyl-5-methylcyclohoxanecarbonyl-D-alanine methyl ester.

4. Preparations according to one of the preceding claims, **characterized in that** they comprise 0.001% by weight to 20% by weight, preferably 0.01% by weight to 10% by weight, in particular 0.1-10% by weight, based on the total weight of the preparations, of one or more skin moisturizing agents.

## Revendications

1. Préparations cosmétiques ou dermatologiques présentant une teneur en ester méthylique de la 2-isopropyl-5-méthyl-cyclohexanecarbonyl-D-alanine et un ou plusieurs agents d'hydratation de la peau à l'exception des solutions à 0,1% et à 0,5% (% en poids) de l'ester méthylique de la 2-isopropyl-5-méthyl-cyclohexanecarbonyl-D-alanine dans des solutions de 10% de propylèneglycol dans 90% d'eau.

2. Préparations selon la revendication 1, **caractérisées en ce que** le ou les agents d'hydratation de la peau est ou sont choisi(s) dans le groupe formé par le glycérol, le méthylpropanediol, l'hexanediol, l'octanediol, la taurine, le chlorure de sodium, l'acide lactique et/ou les lactates, en particulier le lactate de sodium, le butylèneglycol, le propylèneglycol, le Biosaccaride Gum-1, l'éthylhexyloxyglycérol, l'acide pyrrolidonecarboxylique, l'acide hyaluronique et l'urée.

3. Préparations selon l'une quelconque des revendications précédentes, **caractérisées en ce qu'**elles contiennent 0,001% en poids à 10% en poids, de préférence 0,01% en poids à 1,0% en poids, en particulier 0,05-0,5% en poids, par rapport au poids total des préparations, d'ester méthylique de la 2-isopropyl-5-méthyl-cyclohexanecarbonyl-D-alanine.

4. Préparations selon l'une quelconque des revendications précédentes, **caractérisées en ce qu'**elles contiennent 0,001% en poids à 20% en poids, de préférence 0,01% en poids à 10% en poids, en particulier 0,1-10% en poids, par rapport au poids total des préparations, d'un ou de plusieurs agent(s) d'hydratation de la peau.
